# EUROPEAN PATENT APPLICATION

(11) **EP 4 545 018 A1**
(43) Date of publication of application: **30.04.2025**
(21) Application number: 24769886.3
(22) Date of filing: 08.03.2024
(51) Int. Cl.: A61B 17/122, A61B 17/128, A61B 17/12

(54) **HEMOSTATIC CLIP ASSEMBLY AND HEMOSTATIC CLIP COMPRISING SAME**

(30) Priority: 10.03.2023 CN 202320441753 U; 10.03.2023 CN 202310225453
(71) Applicant: Jiangsu Vedkang Medical Science and Technology Co., Ltd., Changzhou, Jiangsu 213149 (CN)
(72) Inventor: MIAO, Donglin, hangzhou, Jiangsu 213149 (CN)
(74) Representative: karo IP
(86) International application number: PCT/CN2024/080849
(87) International publication number: WO 2024/188193

(57) **Abstract**

The present application discloses a hemostatic clip assembly and a hemostatic clip comprising same, including a sleeve, a two-piece clamp, and a pulling device connected to the two-piece clamp, where the two-piece clamp includes a fixing member and two clamping pieces connected through the fixing member and disposed opposite to each other, and each clamping piece includes a first clamping section close to a distal end and a second clamping section close to a proximal end; and the fixing member penetrates through the second clamping section. Each second clamping section is provided with a first locking mechanism and a second locking mechanism disposed relative to each other, the sleeve is provided with a third locking mechanism, after the pulling device is disengaged from the second locking mechanism, the two first locking mechanisms move away from each other, and the first locking mechanisms are matched with the third locking mechanism to fix the two-piece clamp and the sleeve. The clamping piece of the present application has a simpler manufacturing process, a joint of the two clamping pieces is reduced in stress, and the clamping piece can be detached and replaced.

## Description

### Technical Field

This application relates to the technical field of medical instruments, and in particular, to a hemostatic clip assembly and a hemostatic clip comprising same.

### Background Technology

A hemostatic clip is a tool that generally adopts the following operations: utilizing mechanical pressure generated by closure of two clamping pieces to compress a bleeding part and block blood flow; and subsequently, separating the clamping pieces through an external control handle, and leaving the clamping pieces inside a human body, thereby achieving the purpose of continuous hemostasis.

According to the hemostatic clip in the prior art, the two clamping pieces are of an integrated structure, and rear ends of the clamping pieces are fixedly connected. In the process of multiple opening and closing of the clamping pieces, a connecting part of the rear ends of the clamping pieces is large in stress, and a certain risk of breakage exists. Secondly, since the rear ends of the clamping pieces are fixedly connected, when the integrated clamping piece is used, a diameter-varied design on the sleeve is required or a deformable structure needs to be mounted on the clamping piece, so that the clamping pieces are locked with the sleeve when being in a closed state. If the sleeve is designed to be of a diameter-varied structure, the mold opening cost may be increased, and the clamping pieces need to cross the diameter-varied part of the sleeve to be locked with the sleeve, the resistance of pulling back the clamping pieces is relatively large, which increases the operation difficulty. If the deformable structure is mounted on the clamping pieces, the specific structure and the deformation mode of the deformable structure need to be additionally designed, resulting in a large change in the original clamping pieces and an increase in production cost.

In addition, a pull rod in the existing hemostatic clip is mostly connected to the clamping piece through a C-shaped matching portion with a C-shaped structure. When the pull rod is separated from the clamping piece, an opening of the C-shaped matching portion is increased, the opened C-shaped matching portion is difficult to restore to a standard C-shaped structure, and the C-shaped matching portion cannot be stably connected to the rear end of the clamping piece again, resulting in poor retrievability of the pull rod.

In the prior art, a clamping piece having a two-piece structure is also adopted, however, due to the unreasonable structural design, the clamping piece is difficult to disengage from the pull rod.

Furthermore, according to the hemostatic clip in the prior art, the two clamping pieces are of an integrated structure, and rear ends of the clamping pieces are fixedly connected. In the process of multiple opening and closing of the clamping pieces, a connecting part of the rear ends of the clamping pieces is large in stress, and a certain risk of breakage exists. Secondly, a proximal end of the integrated clamping piece cannot move freely, a new connecting structure cannot be processed on the clamping piece, and the pull rod can only be connected to the proximal end of the clamping piece through the C-shaped matching portion. When the pull rod is separated from the clamping piece, an opening of the C-shaped matching portion is increased, the opened C-shaped matching portion is difficult to restore to a standard C-shaped structure, and the C-shaped matching portion cannot be stably connected to the rear end of the clamping piece again, resulting in poor retrievability of the pull rod. Moreover, another deformable structure needs to be mounted, so that the clamping piece is locked with sleeve when being in a closed state, which increases the production cost.

In addition, in the traditional structure, the pull rod is connected to the proximal end of the clamping piece, the locking of the clamping piece and the sleeve as well as the separation of the pull rod and the clamping piece need to overcome respective resistance, that is, the clamping piece can only be released by overcoming the double resistance, and it is difficult to control the timing of locking the clamping piece and the sleeve, the sequential order of the locking of the clamping piece and the sleeve as well as the separation of the clamping piece and the pull rod can only be ensured through accurate structural design and material selection, and otherwise, a phenomenon that the clamping piece is not locked with the sleeve after the pull rod is disengaged may occur, and therefore, the requirement for processing precision is relatively high.

### Summary of the Invention

In order to solve the technical problems that in the prior art, two clamping pieces in the hemostatic clip are of an integrated structure, the clamping piece has a risk of breakage, a locking structure of the clamping piece and a sleeve is complex, and the cost is relatively high, the present application provides a hemostatic clip assembly and a hemostatic clip comprising same to solve the above problems.

The present application provides a hemostatic clip assembly, including a sleeve, a two-piece clamp, and a pulling device connected to the two-piece clamp, where the two-piece clamp and the pulling device are at least partially accommodated in the sleeve, the two-piece clamp includes a fixing member and two clamping pieces connected through the fixing member and disposed opposite to each other, and each clamping piece includes a first clamping section close to a distal end and a second clamping section close to a proximal end; and the fixing member penetrates through the second clamping section.

Each second clamping section is provided with a first locking mechanism and a second locking mechanism disposed relative to each other, and one end of the pulling device is separably connected to the two-piece clamp through the second locking mechanism and enables the two first locking mechanisms to be close to each other.

The sleeve is provided with a third locking mechanism, in a process that the pulling device is disengaged from the second locking mechanism, the two-piece clamp moves towards the proximal end under an action of the pulling device, and the sleeve forces the two first clamping sections to be gradually close to each other; and after the pulling device is disengaged from the second locking mechanism, the two first locking mechanisms move away from each other, and the first locking mechanisms are matched with the third locking mechanism to fix the two-piece clamp and the sleeve.

Further, the fixing member is a pin shaft, and the second clamping section is provided with a shaft hole through which the pin shaft passes.

Further, at least one end of an axial direction of the pin shaft is provided with a shielding cap, and a diameter of the shielding cap is greater than a diameter of the shaft hole.

Further, one end of the axial direction of the pin shaft is provided with a shielding cap, and the other end of the axial direction of the pin shaft is a flaring design.

Further, the third locking mechanism is a clamping groove, and the first locking mechanism is a clamping hook.

Further, the second locking mechanism is located between the first locking mechanism and the fixing member.

Further, the pulling device includes a rod portion and a connecting portion which are connected to each other, and the connecting portion is matched with the second locking mechanism, so that the pulling device is connected to the two-piece clamp; and the connecting portion can be deformed and disengaged from the second locking mechanism under the action of a pulling force towards the proximal end.

Further, the pulling device is composed of two pulling plates that sandwich the two second clamping sections, and each pulling plate includes one rod portion and one connecting portion. Further, the second locking mechanism is a matching hole, and the connecting portion includes an insertion plate inserted into the matching hole.

Further, the connecting portion further includes a limiting plate extending towards a direction of the fixing member, the limiting plate is provided with an opening and the opening faces towards the fixing member, and the fixing member is in clearance fit with the limiting plate.

Further, the insertion plates of the two pulling plates are arranged in a parallel and opposite arrangement, in a V-shaped arrangement, in a herringbone arrangement or in a splayed arrangement in the matching hole.

Further, the hemostatic clip assembly further includes a connecting shaft axially limited to the proximal end of the sleeve, where the connecting shaft is provided with a central shaft through which the pulling device passes, and the pulling device can drive the connecting shaft to move towards a direction of the proximal end of the sleeve, so that the connecting shaft is separated from the sleeve.

The present application further provides a hemostatic clip, including a handle assembly and the above hemostatic clip assembly, where the handle assembly drives a pulling device in the hemostatic clip assembly to reciprocate.

According to another aspect of the present application, a two-piece clamp is further provided, including a fixing member and two clamping pieces disposed opposite to each other and connected through the fixing member, where each clamping piece includes a clamping arm section and a clamping tail section extending from a distal end to a proximal end, the clamping arm section includes a first clamping section close to the distal end and a second clamping section close to the proximal end, the fixing member connects respective ends of the two clamping tail sections close to the second clamping section, one end of the clamping tail section away from the second clamping section is provided with a first locking mechanism, the clamping tail section further includes a second locking mechanism disposed between the fixing member and the first locking mechanism, and one end of the pulling device in the hemostatic clip is detachably connected to the two-piece clamp through the second locking mechanism; and in a natural state, respective ends of the two clamping tail sections away from the second clamping section are separated, and the distal ends of the two first clamping sections form an opening.

Further, the fixing member is a pin shaft, and one end of the clamping tail section close to the second clamping section is provided with a shaft hole through which the pin shaft passes.

Further, the second locking mechanism is a matching hole, and an edge of one end of the matching hole away from the second clamping section is a straight line edge.

Further, an inner wall of one end of the matching hole away from the second clamping section is in transition with an outer side surface of the clamping tail section through a rounded corner.

Further, the first locking mechanism is a clamping hook matched with a sleeve in the hemostatic clip.

Further, the second clamping section is arc-shaped.

The present application further provides a hemostatic clip, including a sleeve, a pulling device, and the above two-piece clamp, where the pulling device is connected to the two-piece clamp, the two-piece clamp and the pulling device are at least partially located in the sleeve, the two-piece clamp moves towards a proximal end under the action of the pulling device, and the sleeve forces the two-piece clamp to be in a closed state; and as the pulling device further moves towards the proximal end, the pulling device is separated from the two-piece clamp, and the sleeve is matched with a first locking mechanism.

Further, the pulling device includes a rod portion and a connecting portion which are connected to each other, the connecting portion is connected to a second locking mechanism, and the connecting portion can be deformed and disengaged from the second locking mechanism under the action of a pulling force.

Further, the connecting portion includes a baffle located at two sides of two clamping tail sections and an insertion plate matched with the second locking mechanism, and the insertion plate is inserted into the second locking mechanism, so that the two clamping tail sections are closed. Further, the pulling device is composed of two pulling plates that sandwich the clamping tail sections, each pulling plate includes a rod portion, a baffle, and an insertion plate, and an arrangement direction of the two pulling plates is the same as an arrangement direction of two clamping pieces.

Further, the two insertion plates are in a parallel arrangement, in a V-shaped arrangement, in a herringbone arrangement or in a splayed arrangement.

Further, the connecting portion further includes a limiting plate extending towards a direction of the fixing member, the limiting plate is provided with an opening and the opening faces towards the fixing member, and the fixing member is in clearance fit with the limiting plate.

Beneficial effects of the present application are as follows:
(1) According to the present application, the clamp is composed of two clamping pieces, the two clamping pieces are connected through a fixing member, and each clamping piece is provided with a first locking mechanism matched with a sleeve is locked with the sleeve through elastic deformation of the clamping piece, and compared with an integrated clamping piece, the manufacturing process of the clamping piece in the present application is simpler, a joint of the two clamping pieces is reduced in stress, and the clamping piece can be detached and replaced.
(2) According to the present application, the clamping piece is provided with a second locking mechanism matched with a pulling device, the second locking mechanism is located between the first locking mechanism and the fixing member, and it is ensured that after the pulling device is disengaged from the second locking mechanism, the first locking mechanism can reach a free state and be fixed with the sleeve, and the two-piece clamp is prevented from being locked in advance.
(3) According to the present application, the pulling device is connected to the clamp through an insertion plate inserted into a matching hole, and compared with a traditional C-shaped matching portion, the insertion plate in the present application is easy to reset after being deformed, and can be recycled for many times.
(4) According to the present application, the pulling device adopts a two-piece structure matched with the clamping piece. The structure is symmetrical, and mounting flexibility is relatively high. In addition, the two-piece pulling device is more conducive to opening of the connecting portion.

### Brief Description of Figures

The present application will be further described below with reference to the accompanying drawings and embodiments.
FIG. 1 is an assembly diagram of a two-piece clamp and a pulling device according to the present application;
FIG. 2 is an enlarged view of a position a in FIG. 1;
FIG. 3 is a side view of FIG. 1;
FIG. 4 is a sectional view taken along A-A in FIG. 3;
FIG. 5 is an enlarged view of a position b in FIG. 4;
FIG. 6 is a front view of the hemostatic clip according to the present application when the two-piece clamp is initially closed;
FIG. 7 is a sectional view taken along B-B in FIG. 6;
FIG. 8 is a sectional view taken along B-B of the hemostatic clip according to the present application from the perspective shown in FIG. 6 when the pulling device is deformed;
FIG. 9 is a schematic diagram of a state in which the hemostatic clip is left in a human body after being released;
FIG. 10 is a schematic diagram of a specific implementation of a pulling device according to the present application;
FIG. 11 is a schematic diagram of a pulling device when an insertion plate is in a V-shaped arrangement according to the present application;
FIG. 12 is a schematic diagram of a pulling device when an insertion plate is in a herringbone arrangement according to the present application;
FIG. 13 is a schematic diagram of a pulling device when an insertion plate is horizontally arranged in alignment according to the present application;
FIG. 14 is a schematic diagram of a pulling device when an insertion plate is in a splayed arrangement according to the present application;
FIG. 15 is a perspective view of the two-piece clamp according to the present application;
FIG. 16 is a perspective view of a clamping piece according to the present application;
FIG. 17 is a front view of the two-piece clamp according to the present application;
FIG. 18 is a front view of the hemostatic clip in an open state according to the present application;
FIG. 19 is a right view of FIG. 18;
FIG. 20 is a sectional view taken along A-A in FIG. 18;
FIG. 21 is an enlarged view of a position a in FIG. 20;
FIG. 22 is a sectional view taken along B-B in FIG. 19;
FIG. 23 is a sectional view taken along A-A of the hemostatic clip according to the present application from the perspective shown in FIG. 18 when the two-piece clamp is initially closed;
FIG. 24 is a schematic diagram of a specific implementation of a pulling plate according to the present application;
FIG. 25 is a schematic diagram of a specific implementation of a pulling plate according to the present application; and
FIG. 26 is a schematic diagram of a specific implementation of a pulling plate according to the present application.

In the figures: 1. two-piece clamp; 101. fixing member; 1011. shielding cap; 102. clamping piece; 1021. first clamping section; 1022. second clamping section; 2. first locking mechanism; 3. sleeve; 301. third locking mechanism; 4. pulling device; 401. pulling plate; 4011. rod portion; 4012. baffle; 4013. insertion plate; 4014. bent plate; 5. shaft hole; 6. arc-shaped section; 7. second locking mechanism; 8. limiting plate; 9. connecting shaft; 901. central shaft; 10. outer tube; 11. inhaul cable; 12. lateral boss; 13. casing.

### Detailed Description of Embodiments

Embodiments of the present application are described in detail below and examples of the embodiments are shown in the accompanying drawings, where the same or similar reference signs always represent the same or similar elements or elements with the same or similar functions. The embodiments described below with reference to the accompanying drawings are exemplary merely for explaining the present application and should not be construed as limiting the present application.

In the present application, in a use state, an end close to a target lesion tissue is a distal end, and an end close to an instrument operator is a proximal end.

### Embodiment 1

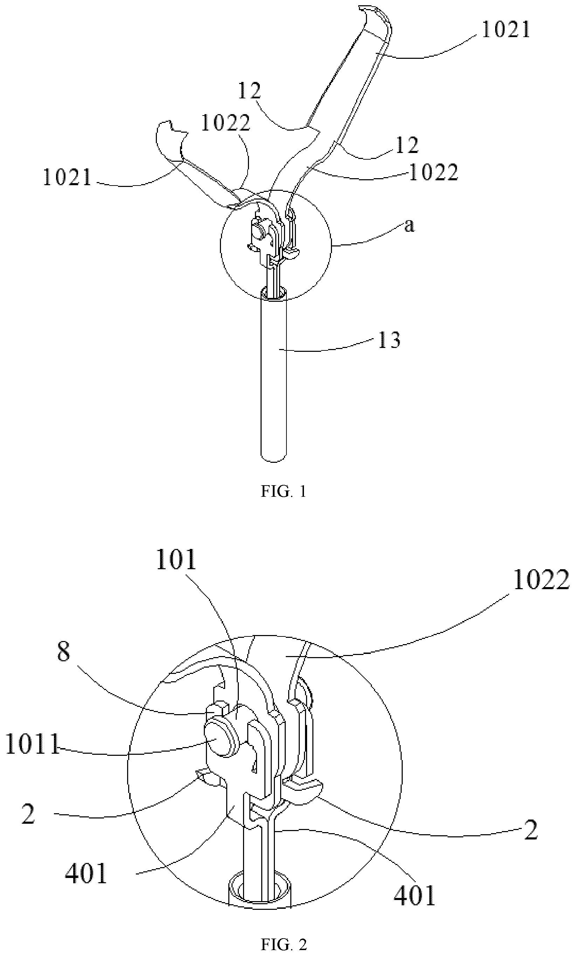

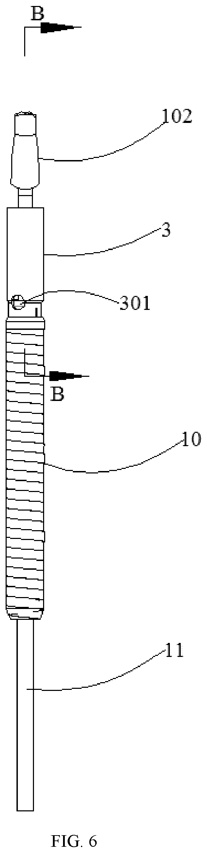

As shown in FIG. 1 to FIG. 6, a hemostatic clip assembly, including a sleeve 3, a two-piece clamp 1, and a pulling device 4 connected to the two-piece clamp 1, where the two-piece clamp 1 and the pulling device 4 are at least partially accommodated in the sleeve 3, the two-piece clamp 1 includes a fixing member 101 and two clamping pieces 102 connected through the fixing member 101 and disposed opposite to each other, and each clamping piece 102 includes a first clamping section 1021 close to a distal end and a second clamping section 1022 close to a proximal end; and the fixing member 101 penetrates through the second clamping section 1022.

Each second clamping section 1022 is provided with a first locking mechanism 2 and a second locking mechanism 7 disposed relative to each other, and one end of the pulling device 4 is separably connected to the two-piece clamp 1 through the second locking mechanism 7 and enables the two first locking mechanisms 2 to be close to each other. The sleeve 3 is provided with a third locking mechanism 301, in a process that the pulling device 4 is disengaged from the second locking mechanism 7, the two-piece clamp 1 moves towards the proximal end under the action of the pulling device 4, and the sleeve 3 forces the two first clamping sections 1021 to be gradually close to each other; and after the pulling device 4 is disengaged from the second locking mechanism 7, the two first locking mechanisms 2 move away from each other, and the first locking mechanisms 2 are matched with the third locking mechanism 301 to fix the two-piece clamp 1 and the sleeve 3.

The second clamping section 1022 is connected to the pulling device 4. The first clamping section 1021 is close to the target lesion or bleeding tissue and is used for clamping human tissue for compression hemostasis. In a free state, the first clamping sections 1021 of the two clamping pieces 102 are in an open state, and when being limited by an inner diameter of the sleeve 3, the first clamping sections 1021 of the two clamping pieces 102 can be closed. That is, when the two-piece clamp 1 is gradually pushed out of the sleeve 3, the distal ends of the two clamping pieces 102 are gradually released by the sleeve 3, the two first clamping sections 1021 are gradually opened, and when the two-piece clamp 1 is pulled back into the sleeve 3, the two first clamping sections 1021 are gradually closed.

In the present application, the first locking mechanisms 2 are close to each other when being bound by the pulling device 4, and can be away from each other in the free state. The first locking mechanisms 2 are located on the second clamping sections 1022, that is, the proximal ends of the second clamping sections 1022 can be away from each other in the free state. The effect can be achieved through the elastic force of the second clamping section 1022, and can also be achieved through an additional reset structure, for example, a reset spring is connected between the two second clamping sections 1022. In this embodiment, the second clamping section 1022 is designed to be arc-shaped, the two clamping pieces 102 are arranged side by side, and the arc-shaped back surfaces of the second clamping sections 1022 are opposite to each other. With respect to the position where the arc-shaped back surfaces of the two second clamping sections 1022 are connected opposite to each other, and a center of curvature of one second clamping section 1022 and a center of curvature of the other second clamping section 1022 are located on two sides of the position where the arc-shaped back surfaces are connected opposite to each other. When the arc-shaped back surfaces of the two second clamping sections 1022 are attached, due to the arc-shaped feature design of the second clamping section 1022 and the lever principle, connecting points of the arc-shaped back surfaces of the two second clamping sections 1022 gradually move towards the distal end. The proximal ends of the two second clamping sections 1022 are opened outwards in the free state, and in a process that the proximal ends of the second clamping sections 1022 are opened outwards, the two first clamping sections 1021 are gradually close to each other, so that the two-piece clamp 1 is in a closed state. In this embodiment, the distal end of the second clamping section 1022 is approximately C-shaped.

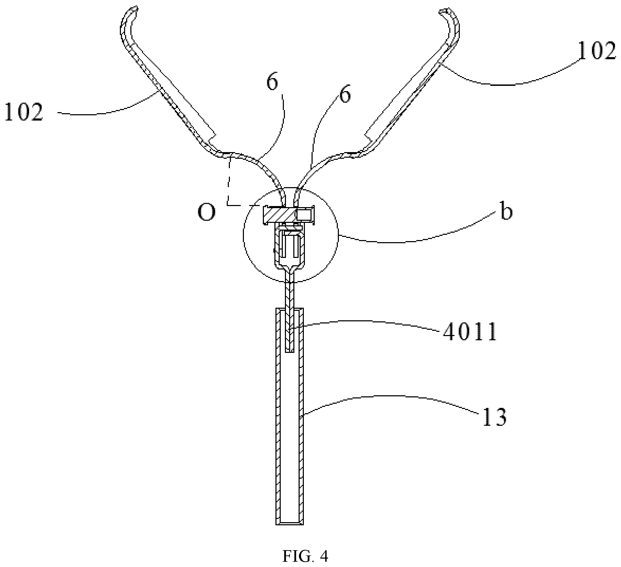

The fixing member 101 is used for connecting the two clamping pieces 102, so that the two clamping pieces 102 can move synchronously. The second clamping section 1022 can be integrally arc-shaped or can be partially arc-shaped, and when the partial area is arc-shaped, an arc-shaped section 6 is preferably disposed at one end close to the first clamping section 1021. Due to the limitation of the inner diameter of the sleeve 3, the second clamping sections 1022 of the two clamping pieces 102 always have parts close to each other in the sleeve 3. When the two first clamping sections 1021 are located outside the sleeve 3, the arc-shaped sections 6 of the two second clamping sections 1022 are partially located outside the sleeve 3, the rear portion of the second clamping section 1022 is limited in the sleeve 3, and the arc-shaped section 6 of the second clamping section 1022 is opened outside the sleeve 3, so that the first clamping section 1021 is opened, and the two-piece clamp 1 is in an open state. When the two second clamping sections 1022 are dragged in the sleeve 3 by the pulling device 4 towards the proximal end, the arc-shaped sections 6 of the two second clamping sections 1022 are attached and extruded inside the sleeve 3, the connecting points of the arc-shaped back surfaces of the two second clamping sections 1022 gradually move towards the first clamping section 1021, and under the action of the lever principle, the proximal end of the second clamping section 1022 gradually tends to be opened. When the pulling device 4 is disengaged from the second locking mechanism 7, the pulling device 4 also releases the constraint on the proximal end of the second clamping section 1022, and the proximal ends of the two second clamping sections 1022 are eventually away from each other, so that the first locking mechanism 2 is matched with the third locking mechanism 301, and in this case, the two first clamping sections 1021 are relatively closed, so that the two-piece clamp 1 is in a closed state. As shown in FIG. 1 and FIG. 4, the arc-shaped section 6 of the clamping piece 102 is located on the second clamping section 1022, taking the clamping piece 102 on the left side of FIG. 4 as an example, a center of curvature of the arc-shaped section 6 on the left side is a point O, and the point O and the second clamping section 1022 on the right side are located on the two sides of the second clamping section 1022 on the left side, respectively, that is, the arc-shaped sections 6 of the two second clamping sections 1022 are bent outwards (i.e., bent in opposite directions).

In this embodiment, the two-piece clamp 1 is composed of two independent clamping pieces 102, and compared with an integrated clamping piece 102 in the prior art, the manufacturing process is simple. The two independent clamping pieces 102 may have a same structure, which facilitates batch production. A connecting part of the two clamping pieces 102 is reduced in stress, so that the breakage phenomenon is avoided. In addition, the clamping piece 102 is provided with the first locking mechanism 2, and the two-piece clamp 1 and the sleeve 3 can be locked through deformation of the clamping piece 102, so that the structure is simplified and the synchronism of actions is improved.

The fixing member 101 is a pin shaft, and the second clamping section 1022 is provided with a shaft hole 5 through which the pin shaft passes. The pin shaft can prevent the two clamping pieces 102 from moving transversely in the sleeve 3, so that the two clamping pieces 102 move in the axial direction more stably, and the acting force of an operator on the pulling device 4 can be relatively stable.

The third locking mechanism 301 may be, but is not limited to, a clamping groove, and the first locking mechanism 2 is a clamping hook matched with the clamping groove. Each second clamping section 1022 is provided with a clamping hook, when the two second clamping sections 1022 are relatively opened, that is, when the first clamping sections 1021 are relatively closed, the clamping hook is matched with the sleeve 3 to fix the two-piece clamp 1 in the sleeve 3. In another embodiment, since the second clamping section 1022 needs to be opened and closed, the fixing member 101 is in clearance fit with the shaft hole 5, that is, the hole diameter of the shaft hole 5 is greater than the diameter of the fixing member 101, and the length of the fixing member 101 needs to satisfy the condition that the clamping hook can reach the clamping groove of the sleeve 3 when the two second clamping sections 1022 are opened. The clamping hooks on the two clamping pieces 102 can be disposed opposite to each other, and can also be arranged diagonally.

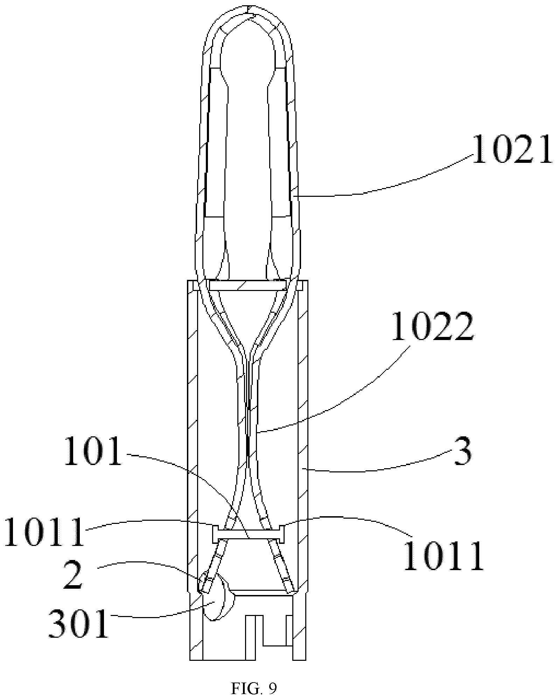

In order to prevent the clamping piece 102 from being disengaged from the pin shaft, preferably, as at least one end of an axial direction of the pin shaft is provided with a shielding cap 1011, and a diameter of the shielding cap 1011 is greater than a diameter of the shaft hole 5. The first locking mechanism 2 is located at the proximal end of the clamping piece 102, and when the second clamping section 1022 is opened, the lateral movement distance of the first locking mechanism 2 is maximum, and it is ensured that the first locking mechanism 2 can be matched with the sleeve 3 (as shown in FIG. 9). In other optional embodiments, one end of the axial direction of the pin shaft can be provided with a shielding cap 1011, and the other end of the axial direction of the pin shaft is a flaring design.

Preferably, the second locking mechanism 7 is located between the first locking mechanism 2 and the fixing member 101, when the pulling device 4 is connected to the second locking mechanism 7, the two first locking mechanisms 2 are forced to be close, and when the pulling device 4 is disengaged from the second locking mechanism 7, the two first locking mechanisms 2 are automatically separated. On the one hand, a relatively large instantaneous release force can be provided for the first locking mechanism 2, so that the first locking mechanism can be quickly matched with the third locking mechanism 301 of the sleeve 3, and on the other hand, the accuracy of the successive movement sequence can be ensured, and the first locking mechanism 2 is prevent from being released in advance.

The pulling device 4 includes a rod portion 4011 and a connecting portion, and the connecting portion is matched with the second locking mechanism 7, so that the pulling device 4 is connected to the two-piece clamp 1; and the connecting portion can be deformed and disengaged from the second locking mechanism 7 under the action of a pulling force towards the proximal end. The connecting portion can be of a claw-shaped structure in a closed state in a free state, and the connecting portion can be deformed and opened under the action of a pulling force.

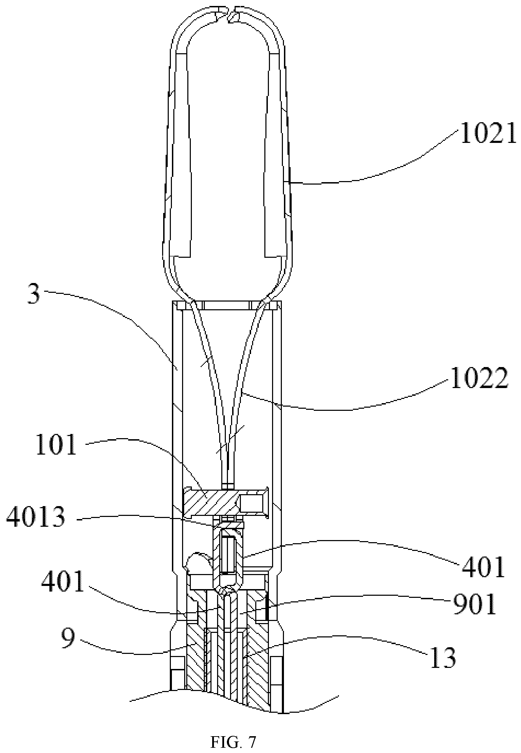

A specific working process is that: when the pulling device 4 is matched with the second locking mechanism 7, the pulling device 4 clamps and pulls the two second clamping sections 1022 close, and when the pulling device 4 pushes the two-piece clamp 1 to move towards the distal end of the sleeve 3, the second clamping section 1022 is gradually pushed out of the sleeve 3, and the two first clamping sections 1021 are freely opened (as shown in FIG. 1). When the two-piece clamp 1 is pulled towards the proximal end of the sleeve 3 to enable the second clamping section 1022 to gradually enter the sleeve 3, under the limitation of the diameter size of the sleeve 3, the two first clamping sections 1021 are close to each other, and the two-piece clamp 1 is in a closed clamping state (as shown in FIG. 7). After the pulling device 4 is separated from the second locking mechanism, the proximal end of the second clamping section 1022 loses pressing force, the proximal ends of the two second clamping sections 1022 are freely opened, and the first locking mechanism 2 is clamped on the third locking mechanism 301 (as shown in FIG. 9).

The width of the first clamping section 1021 is greater than the width of the second clamping section 1022, so that a lateral boss 12 is formed at the proximal end of the first clamping section 1021. When the two-piece clamp 1 is in a closed state, the second clamping section 1022 is at least partially located inside the sleeve 3, the first clamping section 1021 is located outside the sleeve 3, and the lateral boss 12 abuts against the distal end of the sleeve 3, thereby preventing the two-piece clamp 1 from continuing to move towards a direction of the proximal end.

In another embodiment, the second clamping section 1022 is arc-shaped, and the longitudinal axis direction of the first clamping section 1021 is substantially linear. When the two-piece clamp 1 is in a closed state, the maximum spacing of the clamping space formed between the two first clamping sections 1021 is not smaller than an outer diameter of the sleeve 3, so that the arc-shaped back surface of the second clamping section 1022 and the distal end of the sleeve 3 can abut against each other, the two-piece clamp 1 is prevented from being further dragged by the pulling device 4, and the two-piece clamp 1 is prevented from being pulled off from the sleeve 3.

### Embodiment 2

The structure of the pulling device 4 in this embodiment is different from that of the pulling device in Embodiment 1. Specifically: the second locking mechanism 7 is a matching hole, and the connecting portion includes an insertion plate 4013 inserted into the matching hole.

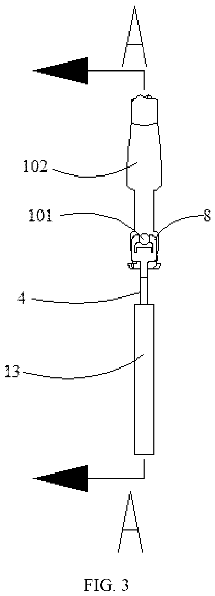

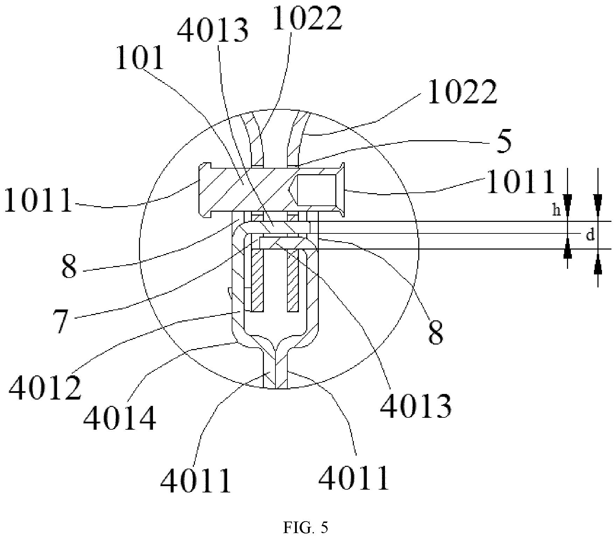

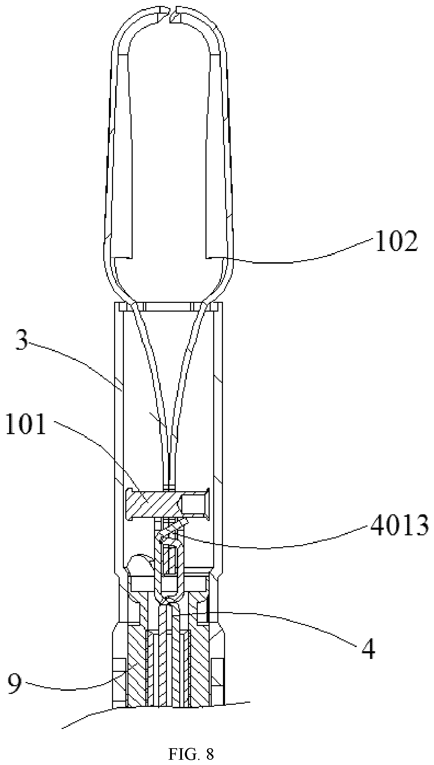

As shown in FIG. 3 to FIG. 5, the two second clamping sections 1022 are provided with matching holes disposed opposite to each other, and the matching holes are located between the pin shaft and the clamping hook. The rod portion 4011 is located at the proximal end of the pulling device 4, and the insertion plate 4013 is connected to the rod portion 4011. When the insertion plate 4013 is matched with the matching hole, an included angle is formed between a surface of the insertion plate 4013 and a surface of the rod portion 4011 (the surface refers to a surface with a larger area). Specifically, the insertion plate 4013 may be formed by laser cutting from the rod portion 4011 and bending to the side, or the insertion plate 4013 and the rod portion 4011 are independent from each other and fixed by welding or riveting, so that the insertion plate 4013 is clamped at the matching hole. As shown in FIG. 5 and FIG. 8, in this embodiment, the matching hole is opened along a horizontal direction, and the insertion plate 4013 is approximately perpendicular to the rod portion 4011. When the pulling device 4 is pulled towards the proximal end, because the two-piece clamp 1 is limited by the sleeve 3, the insertion plate 4013 is gradually pulled into a vertical shape until the insertion plate is pulled out of the matching hole.

Preferably, a wall surface of one end of the matching hole away from the first clamping section 1021 is a flat surface, and an inner wall of the matching hole is in smooth transition with the outer side wall, so as to pull out the insertion plate 4013 more labor-saving. The matching hole can be a rectangular hole, a waist-shaped hole, a triangular hole and the like.

Two insertion plates 4013 are disposed, and the two insertion plates 4013 are inserted from the two sides of the matching hole. A baffle 4012 is further connected between the insertion plate 4013 and the rod portion 4011, the baffle 4012 can press the two second clamping sections 1022, and when the pulling device 4 is pulled towards the proximal end and being subjected to resistance, the baffles 4012 on the two sides are opened towards the two sides. In this embodiment, the two baffles 4012 can be connected to a same rod portion 4011, a size of the matching hole cannot be too large, and if a height h of the matching hole is too large, a gap is formed between the insertion plate 4013 and an inner side surface of the matching hole. When the pulling device 4 is pushed and pulled, the two-piece clamp 1 cannot respond to an action of the pulling device 4 in time. Preferably, when the matching hole is matched with two insertion plates 4013 at the same time, the height h of the matching hole is slightly larger than a sum of thicknesses d of the two insertion plates 4013 (as shown in FIG. 5), so that the two insertion plates 4013 are attached to the inner side surface of the matching hole.

### Embodiment 3

In Embodiment 2, the two baffles 4012 are connected to the same rod portion 4011, the baffle 4012 and the insertion plate 4013 are difficult to open laterally, and the rod portion 4011 and the baffle 4012 are difficult to be integrally formed. If the rod portion and the baffle are fixedly connected in the later period, a connecting part of the rod portion and the baffle is easy to be disconnected. To this end, this present embodiment makes the following improvements:
As shown in FIG. 2, FIG. 3 and FIG. 10, the pulling device 4 is composed of two pulling plates 401 that sandwich the two second clamping sections 1022, and each pulling plate 401 includes one rod portion 4011, one baffle 4012, and one insertion plate 4013. The arrangement direction of the two pulling plates 401 is parallel to the axial direction of the fixing member 101. When the pulling plate 401 is pulled, the baffle 4012 and the insertion plate 4013 can be bent along a thin wall direction of the pulling plate 401, and the bending difficulty is low, which facilitate separation of the pulling device 4 and the two-piece clamp 1.

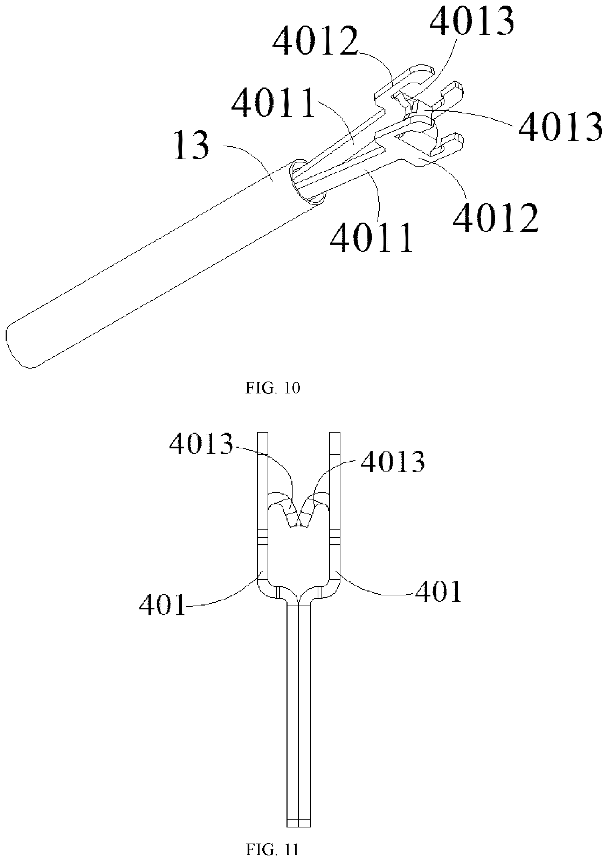

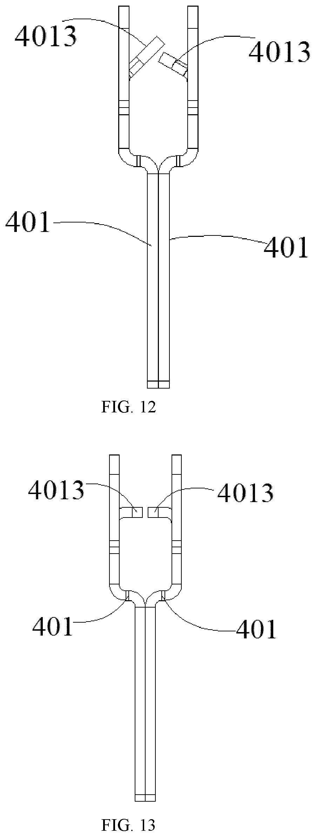

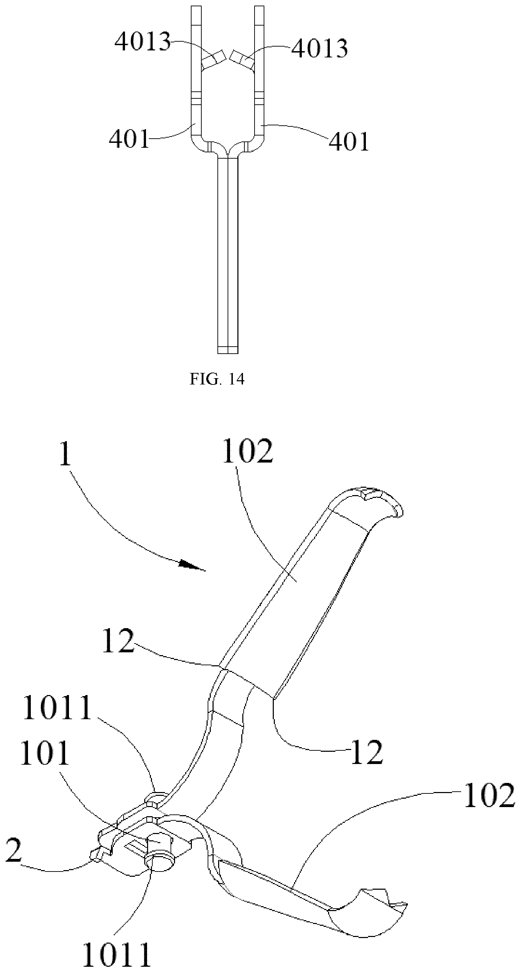

In this embodiment, the pulling device 4 is composed of two independent pulling plates 401. The rod portion 4011, the baffle 4012 and the insertion plate 4013 on the pulling plate 401 can be integrally formed, and a breakage phenomenon does not occur during bending deformation. When the pulling device 4 is ready to be disengaged from the second locking mechanism 7, the distal ends of the two pulling plates 401 can be freely opened. The two insertion plates 4013 are inserted into the matching hole to apply a pulling acting force to the two-piece clamp 1. The two insertion plates 4013 can be in a parallel and opposite arrangement, in a V-shaped arrangement (as shown in FIG. 11), in a herringbone arrangement (as shown in FIG. 12) or in a splayed arrangement (as shown in FIG. 14) in the matching hole. The parallel and opposite arrangement includes a horizontal alignment arrangement (as shown in FIG. 13) and a stacking arrangement (as shown in FIG. 10). When the pulling device 4 is not pulled, the two insertion plates 4013 are abutted against the matching hole and cannot be disengaged from the matching hole.

The pulling plate 401 can be in a straight plate shape, and in this case, the distance between the two baffles 4012 is equal to the distance between the two rod portions 4011. Since the two baffles 4012 cannot be attached to each other, the two pulling plates 401 need to be arranged obliquely (as shown in FIG. 10). In this embodiment, the pulling plate 401 is in a bent shape, and as shown in FIG. 12 and FIG. 5, the rod portion 4011 of the pulling plate 401 is connected to the baffle 4012 through a bent plate 4014. In this case, the two rod portions 4011 are attached to each other, and the two rod portions 4011 can be partially connected. The design of the bent plate 4014 also facilitates deformation and opening of the baffle 4012.

### Embodiment 4

On the basis of Embodiment 2 and Embodiment 3, the connecting portion further includes a limiting plate 8 extending towards a direction of the fixing member 101, the limiting plate 8 is provided with an opening and the opening faces towards the fixing member 101, and the fixing member 101 is in clearance fit with the limiting plate 8, as shown in FIG. 2. The limiting plate 8 is matched with the fixing member 101 to enable the symmetry axes of the pulling device 4 and the two-piece clamp 1 to be located on the same straight line, so that the pulling device 4 is prevented from inclining. In addition, the limiting plate 8 is located between the second clamping section 1022 and the shielding cap 1011 (or an end flaring of the fixing member 101), and the limiting plate 8 is matched with the shielding cap 1011 and the flaring for use. The shielding cap 1011 and the end flaring of the fixing member 101 are a maximum limiting position where the second clamping section 1022 expands outwards in a natural state, and the limiting plate 8 is a limiting position where the second clamping section 1022 expands outwards when the pulling device 4 is connected to the second locking mechanism 7. Meanwhile, the effect of preventing the fixing member 101 from moving left and right transversely is also achieved.

### Embodiment 5

On the basis of the above embodiments, the hemostatic clip assembly further includes a connecting shaft 9 axially limited to a rear end of the sleeve 3, where the connecting shaft 9 is provided with a central shaft 901 through which the pulling device 4 passes, and the pulling device 4 can drive the connecting shaft 9 to move towards the proximal end of the sleeve 3, so that the connecting shaft 9 is separated from the sleeve 3. The connecting shaft 9 conceals a connecting section of the pulling device 4 and the two-piece clamp 1 in the sleeve 3. The connecting shaft 9 is generally of a tubular structure, and in order to avoid changing the structure of the connecting shaft 9, the casing 13 can be fixed at the proximal end of the pulling device 4, and the casing 13 is matched with the central shaft 901.

A contact surface of the sleeve 3 and the connecting shaft 9 can be a reducing cylindrical surface, the connecting shaft 9 is extruded at the proximal end of the sleeve 3, and under the action of a pulling force of the pulling device 4, the connecting shaft 9 is contracted and deformed to be disengaged from the proximal end of the sleeve 3. In other embodiments, a barb abutting against an end surface of the connecting shaft 9 can also be disposed at the proximal end of the sleeve 3, the barb supports a rear end surface of the connecting shaft 9 to prevent the connecting shaft 9 from being disengaged. When the pulling device 4 drives the connecting shaft 9 to move towards a direction of the proximal end of the sleeve 3, the barb is extruded and straightened, and the connecting shaft 9 is disengaged from the proximal end of the sleeve 3.

### Embodiment 6

A hemostatic clip includes a handle assembly and the above hemostatic clip assembly, where the handle assembly drives a pulling device 4 in the hemostatic clip assembly to reciprocate. The handle assembly specifically includes an outer tube 10 and an inhaul cable 11, one end of the connecting shaft 9 is connected to the sleeve 3, the other end of the connecting shaft is connected to the outer tube 10, and the inhaul cable 11 penetrates through the outer tube 10 to be connected to the pulling device 4. A handle is mounted at a proximal end of the inhaul cable 11, and a reciprocating motion of the pulling device 4 is controlled by the handle. The outer tube 10 is generally a spring tube with a better flexibility.

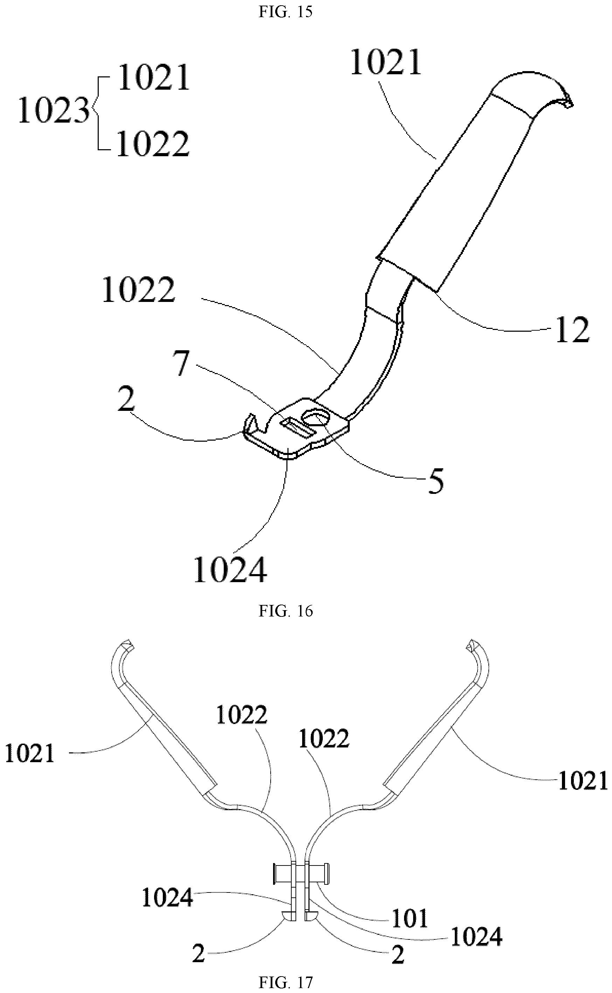

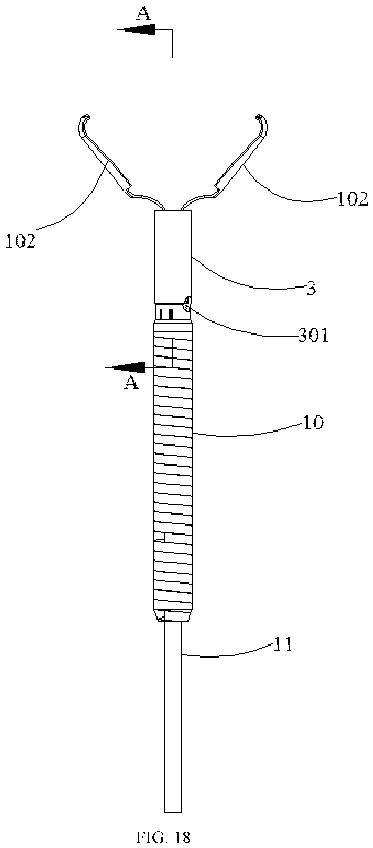

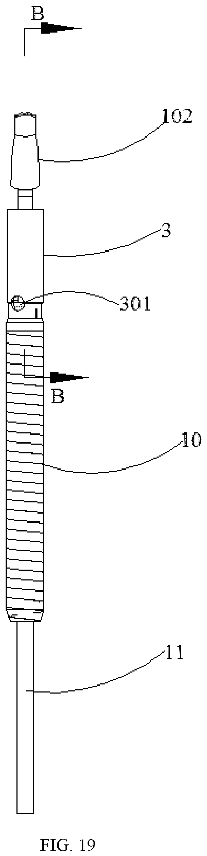

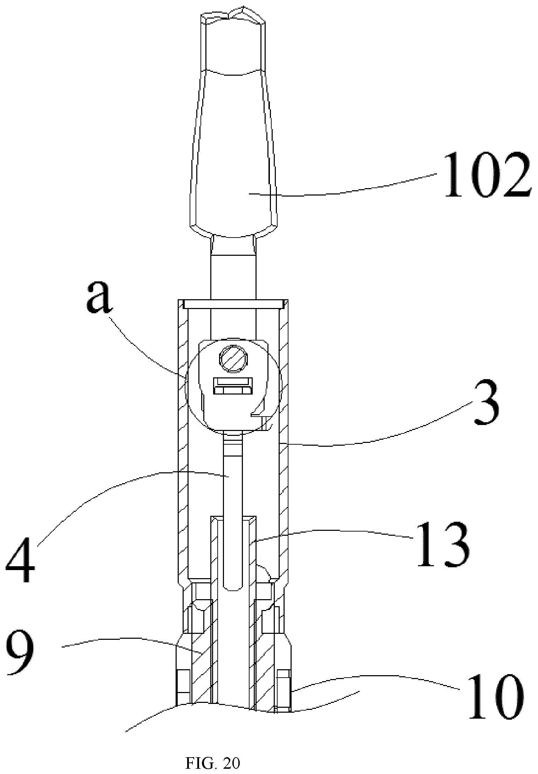

Referring to FIG. 15 to FIG. 22, according to another aspect of the present application, a two-piece clamp 1 is further provided, including a fixing member 101 and two clamping pieces 102 disposed opposite to each other and connected through the fixing member 101, where each clamping piece 102 includes a clamping arm section 1023 and a clamping tail section 1024 extending from a distal end to a proximal end, the clamping arm section 1023 includes a first clamping section 1021 close to the distal end and a second clamping section 1022 close to the proximal end, the fixing member 101 connects respective ends of the two clamping tail sections 1024 close to the second clamping section 1022, one end of the clamping tail section 1024 away from the second clamping section 1022 is provided with a first locking mechanism 2, and after being successfully clamped, the first locking mechanism 2 is matched with the sleeve 3 in the hemostatic clip, so that the clamping piece 102 and the sleeve 3 are locked and left in the human body together. The clamping tail section 1024 further includes a second locking mechanism 7 disposed between the fixing member 101 and the first locking mechanism 2, and one end of the pulling device in the hemostatic clip is detachably connected to the two-piece clamp 1 through the second locking mechanism 7; and in a natural state, respective ends of the two clamping tail sections 1024 away from the second clamping section 1022 are separated, and the distal ends of the two first clamping sections 1021 form an opening.

The fixing member 101 is used for connecting the two clamping pieces 102, so that the two clamping pieces 102 can move synchronously. The clamping tail section 1024 and the first clamping section 1021 are both opened in a natural state, a non-natural state of the clamping tail section 1024 is limited by the cooperation of the pulling device and the second locking mechanism 7, and a non-natural state of the first clamping section 1021 is that the clamping piece 102 is retracted into the sleeve 3 to be limited by an inner diameter of the sleeve 3. **In** the present application, the two clamping pieces 102 are independent of each other. Compared with a traditional clamping piece 102 structure, the clamping piece 102 in the present application is additionally provided with a clamping tail section 1024, and a first locking mechanism 2, a second locking mechanism 7, and a mounting fixing member 101 are disposed on the clamping tail section 1024, the function integration degree is high, and no additional mounting matching structure is needed. In addition, in the present application, the second locking mechanism 7 is not located at the most proximal end of the clamping piece 102, but is located between the fixing member 101 and the first locking mechanism 2. Under the condition that the pulling device is connected with the second locking mechanism 7, the clamping tail section 1024 is always in a closed state, the first locking mechanism 2 cannot be started, and the first locking mechanism 2 can be started only after the pulling device is disengaged from the second locking mechanism 7, so that the locking timing can be accurately controlled, it is not necessary to control the sequential order of the separation of the pulling device as well as the locking of the sleeve 3 and the clamping piece 102 through mechanical structure design and special selection of a material, and the production cost is reduced.

### Embodiment 7

As shown in FIG. 15 to FIG. 18, a two-piece clamp 1 includes a fixing member 101 and two clamping pieces 102 disposed opposite to each other and connected through the fixing member 101. The fixing member 101 is a pin shaft, and the two clamping pieces 102 have the same structure and are symmetrically arranged. Each clamping piece 102 includes a clamping arm section 1023 and a clamping tail section 1024 extending from a distal end to a proximal end, the clamping arm section 1023 includes a first clamping section 1021 close to the distal end and a second clamping section 1022 close to the proximal end, the fixing member 101 connects respective ends of the two clamping tail sections 1024 close to the second clamping section 1022, the clamping tail section 1024 is provided with a shaft hole 5, the fixing member 101 penetrates through the shaft holes 5 of the two clamping tail sections 1024, the fixing member 101 can prevent the two clamping pieces 102 from moving transversely in the sleeve 3, so that the two clamping pieces 102 move in the axial direction more stably, and the acting force of an operator on the pulling device can be relatively stable. One end of the clamping tail section 1024 away from the second clamping section 1022 is provided with a first locking mechanism 2, and after being successfully clamped, the first locking mechanism 2 is matched with the sleeve 3 in the hemostatic clip, so that the clamping piece 102 and the sleeve 3 are locked and left in the human body together. The clamping tail section 1024 further includes a second locking mechanism 7 disposed between the fixing member 101 and the first locking mechanism 2, and one end of the pulling device in the hemostatic clip is detachably connected to the two-piece clamp 1 through the second locking mechanism 7; and in a natural state, respective ends of the two clamping tail sections 1024 away from the second clamping section 1022 are separated, and the distal ends of the two first clamping sections 1021 form an opening.

In the present application, the first locking mechanisms 2 are close to each other when being bound by the pulling device, and can be away from each other in the natural state. The first locking mechanisms 2 are located on the clamping tail sections 1024, and therefore, the proximal ends of the clamping tail sections 1024 can be away from each other in the natural state. The effect can be achieved through the elastic force of the clamping piece 102, and can also be achieved through an additional reset structure, for example, a reset spring is connected between the two clamping pieces 102. In this embodiment, the second clamping section 1022 is designed to be arc-shaped, the two clamping pieces 102 are arranged side by side, and the arc-shaped back surfaces of the second clamping sections 1022 are opposite to each other. When the arc-shaped back surfaces of the two second clamping sections 1022 are attached, a closing process of the two-piece clamp 1 is taken as an example, due to the arc-shaped feature design of the second clamping section 1022 and the lever principle, connecting points of the arc-shaped back surfaces of the two second clamping sections 1022 gradually move towards a direction of the first clamping section 1021. The two clamping tail sections 1024 are opened outwards in a free state, and in a process that the clamping tail sections 1024 are opened outwards, the two first clamping sections 1021 are gradually close to each other, so that the two-piece clamp 1 is in a closed state. In this embodiment, the second clamping section 1022 is approximately C-shaped (as shown in FIG. 17).

In order to prevent the pin shaft (fixing member 101) from being disengaged from the shaft hole 5, preferably, as at least one end of an axial direction of the pin shaft is provided with a shielding cap 1011, and a diameter of the shielding cap 1011 is greater than a diameter of the shaft hole 5. The first locking mechanism 2 is located at the proximal end of the clamping piece 102, and when the clamping tail section 1024 is opened, the lateral movement distance of the first locking mechanism 2 is maximum, and it is ensured that the first locking mechanism 2 can be matched with the sleeve 3 (as shown in FIG. 9). In other optional embodiments, one end of the axial direction of the pin shaft can be provided with a shielding cap 1011, and the other end of the axial direction of the pin shaft is a flaring design.

The first locking mechanism 2 may be, but is not limited to, a clamping hook. When the two clamping tail sections 1024 are relatively opened, that is, when the first clamping sections 1021 are relatively closed, the clamping hook is matched with the sleeve 3 to fix the two-piece clamp 1 in the sleeve 3. In another embodiment, since the clamping tail section 1024 needs to be opened and closed, the fixing member 101 is in clearance fit with the shaft hole 5, that is, the hole diameter of the shaft hole 5 is greater than the diameter of the fixing member 101, and the length of the fixing member 101 needs to satisfy the condition that the clamping hook can reach the sleeve 3 of the hemostatic clip when the two clamping tail sections 1024 are opened. The clamping hooks on the two clamping pieces 102 can be disposed opposite to each other, and can also be arranged diagonally.

The second locking mechanism 7 may be, but is not limited to, a matching hole. The pulling device 4 is inserted into the matching hole to achieve connection between the pulling device and the matching hole, the matching hole penetrates through a thickness direction of the clamping piece 102, and the pulling device 4 is pulled towards the direction of the proximal end of the sleeve 3, so that the pulling device 4 can be disengaged from the matching hole. The matching hole can be a waist-shaped hole, a square hole, a triangular hole or other holes allowing the pulling device 4 to be inserted. In order to increase a matching contact surface between the pulling device 4 and the matching hole, preferably, an edge of one end of the matching hole away from the second clamping section 1022 is a straight line edge. When the pulling device 4 is disengaged from the matching hole, friction is generated between the pulling device 4 and the edge of the matching hole, and the pulling device 4 is prevented from being disengaged from the matching hole. To this end, an inner wall of one end of the matching hole away from the second clamping section 1022 is in transition with an outer side surface of the clamping tail section 1024 through a rounded corner, so that the disengagement of the pulling device 4 is smoother.

### Embodiment 8

This embodiment provides a hemostatic clip, as shown in FIG. 7, FIG. 9, and FIG. 18 to FIG. 23, including a sleeve 3, a pulling device, and the above two-piece clamp 1. The pulling device includes a pulling device 4 connected to the two-piece clamp 1, and the pulling device 4 can specifically be a pull rod. The two-piece clamp 1 and the pulling device 4 are at least partially located in the sleeve 3, the two-piece clamp 1 moves towards a proximal end under the action of the pulling device 4, and the sleeve 3 forces the two-piece clamp 1 to be in a closed state; and as the pulling device 4 further moves towards the proximal end, the pulling device 4 is separated from the two-piece clamp 1, and the sleeve 3 is matched with a first locking mechanism 2. When the first locking mechanism 2 is a clamping hook, a third locking mechanism 301 is correspondingly disposed on the sleeve 3.

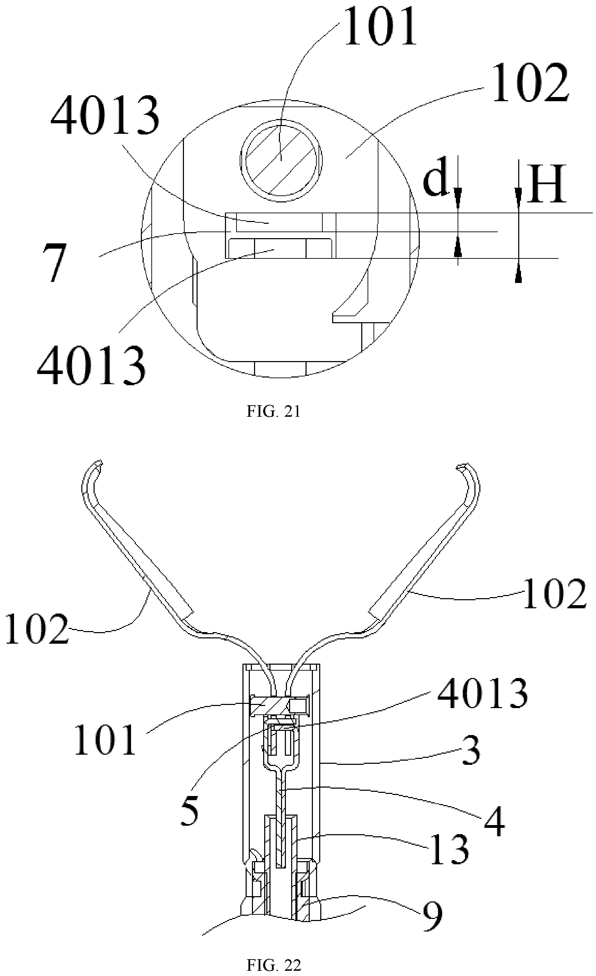

The two clamping pieces 102 are closed under the action of the sleeve 3 and the pulling device 4. When the pulling device 4 is connected to the second locking mechanism 7, the pulling device 4 pulls the two clamping tail sections 1024 close, and when the pulling device 4 pushes the two-piece clamp 1 to move towards the distal end, the first clamping section 1021 moves to the outside of the sleeve 3, and the two first clamping sections 1021 are freely opened (as shown in FIG. 22). When the two-piece clamp 1 is reversely pulled to enable the first clamping section 1021 to enter the sleeve 3, under the limitation of the diameter size of the sleeve 3, the two first clamping sections 1021 are close to each other, and the two-piece clamp 1 is in a closed clamping state (as shown in FIG. 7). After the pulling device 4 is disengaged from the second locking mechanism 7, the clamping tail section 1024 loses pressing force, the two clamping tail sections 1024 are freely opened, and the first locking mechanism 2 is opened to match with the third locking mechanism 301 (as shown in FIG. 9).

The width of the first clamping section 1021 is greater than the width of the second clamping section 1022, so that a lateral boss 12 is formed at the proximal end of the first clamping section 1021. When the two-piece clamp 1 is in a closed state, the second clamping section 1022 and the clamping tail section 1024 are located inside the sleeve 3, the first clamping section 1021 is located outside the sleeve 3, and the lateral boss 12 abuts against a front end surface of the sleeve 3, thereby preventing the two-piece clamp 1 from continuing to move towards a direction of the proximal end of the sleeve 3.

The pulling device 4 is connected to the clamping piece 102, and the two clamping tail sections 1024 can be pressed against each other. The pulling device 4 may include a rod portion 4011 and a connecting portion, the connecting portion is connected to the second locking mechanism 7, and the connecting portion can be deformed and disengaged from the second locking mechanism 7 under the action of a pulling force. The rod portion 4011 is located at the proximal end of the pulling device 4 and is connected to an operating end, and the connecting portion is located at the distal end of the pulling device 4 and is used for matching with the second locking mechanism 7. The connecting portion can be of a claw-shaped structure in a closed state in a natural state, and the connecting portion can be deformed and opened under the action of a pulling force. In this embodiment, when the second locking mechanism 7 is a matching hole, the specific structure of the connecting portion is as follows:
the connecting portion includes a baffle 4012 located at two sides of two clamping tail sections 1024 and an insertion plate 4013 matched with the second locking mechanism 7, and the insertion plate 4013 is inserted into the second locking mechanism 7, so that the two clamping tail sections 1024 are closed.

As shown in FIG. 16, FIG. 21 and FIG. 26, the two clamping tail sections 1024 are provided with matching holes disposed opposite to each other. The pulling device 4 includes a rod portion 4011, a baffle 4012 located at two sides of two clamping tail sections 1024 and an insertion plate 4013 matched with the matching hole. The two baffles 4012 clamp the two clamping tail sections 1024 close to each other, and when the insertion plate 4013 is inserted into the matching hole, the two clamping tail sections 1024 are attached. The insertion plate 4013 is deformed and disengaged from the matching hole under the action of a pulling force of the pulling device 4, so that the two clamping tail sections 1024 are in a free state.

When the insertion plate 4013 is matched with the matching hole, an included angle is formed between a surface of the insertion plate 4013 and a surface of the rod portion 4011 (the surface refers to a surface with a larger area). Specifically, the insertion plate 4013 may be formed by laser cutting from the rod portion 4011 and bending to the side, or the insertion plate 4013 and the rod portion 4011 are independent from each other and fixed by welding or riveting, so that the insertion plate 4013 is clamped at the matching hole. As shown in FIG. 7, FIG. 8 and FIG. 15, in this embodiment, the matching hole is a rectangular hole and penetrates along a horizontal direction. The insertion plate 4013 is approximately perpendicular to the rod portion 4011. When the pulling device 4 is pulled towards the proximal end of the sleeve 3, because the two-piece clamp 1 is limited by the sleeve 3, the baffles 4012 on the two sides are opened towards the two sides, and meanwhile, the insertion plate 4013 is gradually pulled into a vertical shape until the insertion plate is pulled out of the matching hole.

**In** this embodiment, the two baffles 4012 can be connected to a same rod portion 4011. Only one insertion plate 4013 may be disposed to be inserted from one side of the matching hole, or two insertion plates 4013 may be disposed, and the two insertion plates 4013 are inserted from two sides of the matching hole. The two insertion plates 4013 are inserted into the matching hole to apply a pulling acting force to the two-piece clamp 1. The two insertion plates 4013 can be in a parallel arrangement, in a V-shaped arrangement (as shown in FIG. 11), in a herringbone arrangement (as shown in FIG. 12) or in a splayed arrangement (as shown in FIG. 14) in the matching hole. The parallel arrangement includes a horizontal alignment arrangement (as shown in FIG. 13) and a stacking arrangement (as shown in FIG. 5). When the pulling device 4 is not pulled, the two insertion plates 4013 are abutted against the matching hole and cannot be disengaged from the matching hole.

A size of the matching hole cannot be too large, and if a height h of the matching hole is too large, a gap is formed between the insertion plate 4013 and an inner side surface of the matching hole. When the pulling device 4 is pushed and pulled, the two-piece clamp 1 cannot feed back an action of the pulling device 4 in time. Preferably, when the matching hole is matched with two mutually stacked insertion plates 4013, the height h of the matching hole is slightly larger than a sum of thicknesses d of the two insertion plates 4013 (as shown in FIG. 21), so that the two insertion plates 4013 are attached to the inner side surface of the matching hole.

### Embodiment 9

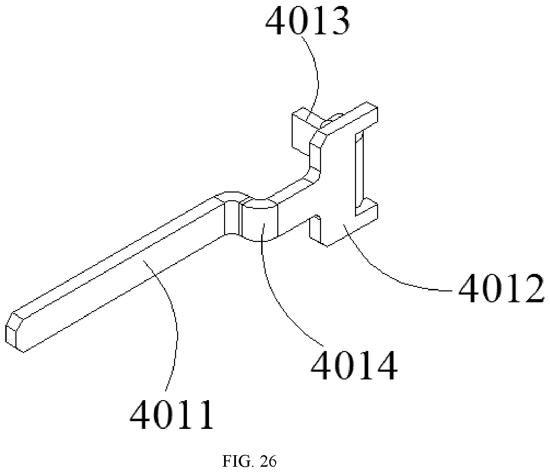

In Embodiment 8, the two baffles 4012 are connected to the same rod portion 4011, the baffle 4012 is difficult to open laterally, and the rod portion 4011 and the baffle 4012 are difficult to be integrally formed. If the rod portion and the baffle are fixedly connected in the later period, a connecting part of the rod portion and the baffle is easy to be disconnected. To this end, this present embodiment makes the following improvements:
As shown in FIG. 7 and FIG. 26, the pulling device 4 is composed of two pulling plates 401 that sandwich the clamping tail sections 1024, each pulling plate 401 includes a rod portion 4011, a baffle 4012, and an insertion plate 4013, and an arrangement direction of the two pulling plates 401 is the same as an arrangement direction of two clamping pieces 102, that is, the two pulling plates 401 sandwich the two clamping tail sections 1024.

In this embodiment, the two pulling plates 401 are independently manufactured. The rod portion 4011, the baffle 4012 and the insertion plate 4013 on the pulling plate 401 can be integrally formed, and a breakage phenomenon does not occur during bending deformation. The pulling plate 401 is of a thin plate-shaped structure. When the pulling device 4 is ready to be disengaged from the two-piece clamp 1, the distal ends of the two pulling plates 401 can be can be opened laterally, and the opening direction is a thickness direction of the pulling plate 401.

The pulling plate 401 can be in a straight plate shape, and in this case, the distance between the two baffles 4012 is equal to the distance between the two rod portions 4011. Since the two baffles 4012 cannot be attached to each other, the two pulling plates 401 need to be arranged obliquely (as shown in FIG. 10). In this embodiment, the pulling plate 401 is in a bent shape, and as shown in FIG. 26, the rod portion 4011 of the pulling plate 401 is connected to the baffle 4012 through a bent plate 4014. In this case, the two rod portions 4011 are attached to each other, and the two rod portions 4011 can be partially connected. The design of the bent plate 4014 also facilitates deformation and opening of the baffle 4012.

### Embodiment 10

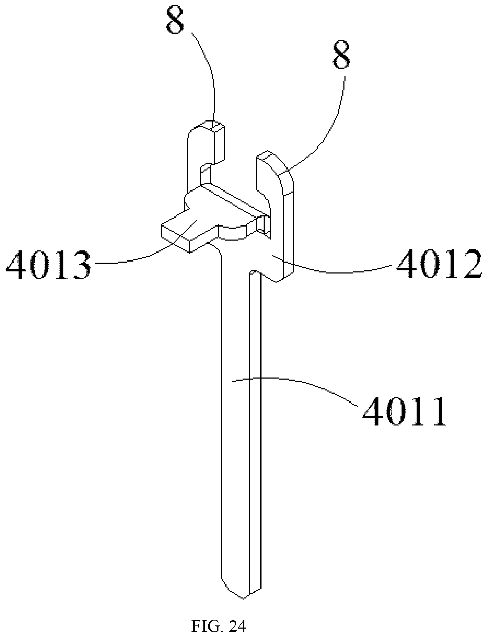

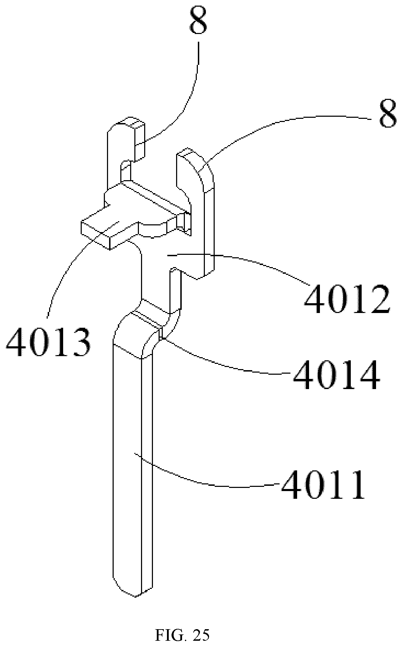

On the basis of Embodiment 9 or Embodiment 10, the baffle 4012 is provided with a limiting plate 8 extending towards a direction of the fixing member 101, the limiting plate 8 is provided with an opening and the opening faces towards the fixing member 101, and the fixing member 101 is in clearance fit with the limiting plate 8. As shown in FIG. 24 and FIG. 25, the limiting plate 8 is located on the two sides of the fixing member 101, so that the symmetry axes of the pulling device 4 and the two-piece clamp 1 to be located on the same straight line, and the pulling device 4 is prevented from inclining. In addition, the limiting plate 8 is located between the clamping tail section 1024 and the shielding cap 1011 (or an end flaring of the fixing member 101), and the limiting plate 8 is matched with the shielding cap 1011 and the flaring for use. The shielding cap 1011 and the end flaring of the fixing member 101 are a maximum limiting position where the clamping tail section 1024 expands outwards in a natural state, and the limiting plate 8 is a limiting position where the clamping tail section 1024 expands outwards when the pulling device 4 is connected to the second locking mechanism 7. Meanwhile, the effect of preventing the fixing member 101 from moving left and right transversely is also achieved.

### Embodiment 5

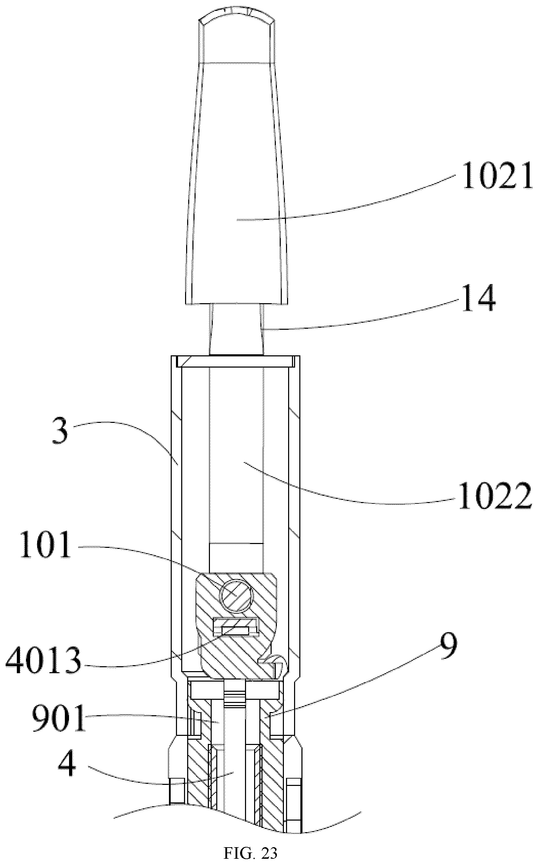

On the basis of the above embodiments, as shown in FIG. 23, the pulling device further includes a connecting shaft 9 axially limited to a proximal end of the sleeve 3, where the connecting shaft 9 is provided with a central shaft 901 through which the pulling device 4 passes, and the pulling device 4 can drive the connecting shaft 9 to move towards a direction of the proximal end of the sleeve 3, so that the connecting shaft 9 is separated from the sleeve 3. The connecting shaft 9 conceals a connecting section of the pulling device 4 and the two-piece clamp 1 in the sleeve 3. The connecting shaft 9 is generally of a tubular structure, the sleeve 3 can be fixed at the proximal end of the pulling device 4, and the casing 13 is matched with the central shaft 901.

A contact surface of the sleeve 3 and the connecting shaft 9 can be a reducing cylindrical surface, the connecting shaft 9 is extruded at the proximal end of the sleeve 3, and under the action of a pulling force of the pulling device 4, the connecting shaft 9 is contracted and deformed to be disengaged from the proximal end of the sleeve 3. In other embodiments, a barb abutting against an end surface of the connecting shaft 9 can also be disposed at the proximal end of the sleeve 3, the barb supports a proximal end surface of the connecting shaft 9 to prevent the connecting shaft 9 from being disengaged. When the pulling device 4 drives the connecting shaft 9 to move towards a direction of the proximal end of the sleeve 3, the barb is extruded and straightened, and the connecting shaft 9 is disengaged from the proximal end of the sleeve 3.

The hemostatic clip usually further includes an outer tube 10 and an inhaul cable 11 arranged in the outer tube 10 in a penetrating manner, two ends of the connecting shaft 9 are respectively connected to the outer tube 10 and the sleeve 3, and the inhaul cable 11 penetrates through the outer tube 10 to be connected to the pulling device 4. A handle is mounted at a rear end of the inhaul cable 11, and a front-back movement of the pulling device 4 is controlled by the handle. The outer tube 10 is generally a spring tube with a better flexibility.

In the description of the present application, it should be understood that the orientation or positional relationship indicated by the terms such as "center", "vertical", "horizontal", "inside", "outside", "axial", etc. are based on the orientation or positional relationship shown in the drawings, and are only for the convenience of describing the present application and simplifying the description, rather than indicating or implying that a device or element referred to should have a specific orientation, be configured and operated in a specific orientation, and therefore cannot be understood as a limitation to the present application.

Furthermore, the terms "first", "second" and the like are used for descriptive purposes only and should not be construed as indication or implication of relative importance.

In this specification, the schematic representation of the term does not necessarily refer to the same embodiment. Moreover, the specific features, structures, materials or characteristics described may be combined in any suitable manner in any one or more embodiments.

Taking the foregoing ideal embodiments of the present application as an inspiration, through the above description, relevant staff can completely make various changes and modifications without deviating from the scope of technical ideas of the present application. The technical scope of the present application is not limited to the contents in the description, but the technical scope thereof must be determined according to the scope of the claims.

## Claims

1. A hemostatic clip assembly, **characterized by** comprising a sleeve (3), a two-piece clamp (1), and a pulling device (4) connected to the two-piece clamp (1), wherein the two-piece clamp (1) and the pulling device (4) are at least partially accommodated in the sleeve (3), the two-piece clamp (1) comprises a fixing member (101) and two clamping pieces (102) connected through the fixing member (101) and disposed opposite to each other, and each clamping piece (102) comprises a first clamping section (1021) close to a distal end and a second clamping section (1022) close to a proximal end; and the fixing member (101) penetrates through the second clamping section (1022);
each second clamping section (1022) is provided with a first locking mechanism (2) and a second locking mechanism (7) disposed relative to each other, and one end of the pulling device (4) is separably connected to the two-piece clamp (1) through the second locking mechanism (7) and enables the two first locking mechanisms (2) to be close to each other;
the sleeve (3) is provided with a third locking mechanism (301), in a process that the pulling device (4) is disengaged from the second locking mechanism (7), the two-piece clamp (1) moves towards the proximal end under an action of the pulling device (4), and the sleeve (3) forces the two first clamping sections (1021) to be gradually close to each other; and
after the pulling device (4) is disengaged from the second locking mechanism (7), the two first locking mechanisms (2) move away from each other, and the first locking mechanisms (2) are matched with the third locking mechanism (301) to fix the two-piece clamp (1) and the sleeve (3).

2. The hemostatic clip assembly according to claim 1, **characterized in that** the fixing member (101) is a pin shaft, and the second clamping section (1022) is provided with a shaft hole (5) through which the pin shaft passes.

3. The hemostatic clip assembly according to claim 2, **characterized in that** the third locking mechanism (301) is a clamping groove, and the first locking mechanism (2) is a clamping hook.

4. The hemostatic clip assembly according to claim 1, **characterized in that** the pulling device (4) comprises a rod portion (4011) and a connecting portion which are connected to each other, and the connecting portion is matched with the second locking mechanism (7), so that the pulling device (4) is connected to the two-piece clamp (1); and the connecting portion can be deformed and disengaged from the second locking mechanism (7) under the action of a pulling force towards the proximal end.

5. The hemostatic clip assembly according to claim 4, **characterized in that** the pulling device (4) is composed of two pulling plates (401) that sandwich the two second clamping sections (1022), and each pulling plate (401) comprises one rod portion (4011) and one connecting portion.

6. The hemostatic clip assembly according to claim 4, **characterized in that** the second locking mechanism (7) is a matching hole, and the connecting portion comprises an insertion plate (4013) inserted into the matching hole.

7. The hemostatic clip assembly according to claim 6, **characterized in that** the insertion plates (4013) of the two pulling plates (401) are in a parallel and opposite arrangement, in a V-shaped arrangement, in a herringbone arrangement or in a splayed arrangement in the matching hole.

8. The hemostatic clip assembly according to claim 4, **characterized in that** the connecting portion further comprises a limiting plate (8) extending towards a direction of the fixing member (101), the limiting plate (8) is provided with an opening and the opening faces towards the fixing member (101), and the fixing member (101) is in clearance fit with the limiting plate (8).

9. The hemostatic clip assembly according to claim 1, **characterized by** further comprising a connecting shaft (9) axially limited to the proximal end of the sleeve (3), wherein the connecting shaft (9) is provided with a central shaft (901) through which the pulling device (4) passes, and the pulling device (4) can drive the connecting shaft (9) to move towards a direction of the proximal end of the sleeve (3), so that the connecting shaft (9) is separated from the sleeve (3).

10. A hemostatic clip, **characterized by** comprising a handle assembly and the hemostatic clip assembly according to any one of claims 1 to 9, wherein the handle assembly drives a pulling device (4) in the hemostatic clip assembly to reciprocate.

11. A two-piece clamp, **characterized by** comprising a fixing member (101) and two clamping pieces (102) disposed opposite to each other and connected through the fixing member (101), wherein each clamping piece (102) comprises a clamping arm section (1023) and a clamping tail section (1024) extending from a distal end to a proximal end, the clamping arm section (1023) comprises a first clamping section (1021) close to the distal end and a second clamping section (1022) close to the proximal end, the fixing member (101) connects respective ends of the two clamping tail sections (1024) close to the second clamping section (1022), one end of the clamping tail section (1024) away from the second clamping section (1022) is provided with a first locking mechanism (2), the clamping tail section (1024) further comprises a second locking mechanism (7) disposed between the fixing member (101) and the first locking mechanism (2), and one end of the pulling device in the hemostatic clip is detachably connected to the two-piece clamp (1) through the second locking mechanism (7); and in a natural state, respective ends of the two clamping tail sections (1024) away from the second clamping section (1022) are separated, and the distal ends of the two first clamping sections (1021) form an opening.

12. The two-piece clamp according to claim 11, **characterized in that** the fixing member (101) is a pin shaft, and one end of the clamping tail section (1024) close to the second clamping section (1022) is provided with a shaft hole (5) through which the pin shaft passes.

13. The two-piece clamp according to claim 11, **characterized in that** the second locking mechanism (7) is a matching hole, and an edge of one end of the matching hole away from the second clamping section (1022) is a straight line edge.

14. The two-piece clamp according to claim 11, **characterized in that** the first locking mechanism (2) is a clamping hook matched with a sleeve (3) in the hemostatic clip.

15. A hemostatic clip, **characterized by** comprising a sleeve (3), a pulling device (4), and the two-piece clamp (1) according to any one of claims 11 to 14, wherein the two-piece clamp (1) is connected to the pulling device (4), the two-piece clamp (1) and the pulling device (4) are at least partially located in the sleeve (3), the two-piece clamp (1) moves towards a proximal end under the action of the pulling device (4), and the sleeve (3) forces the two-piece clamp (1) to be in a closed state; and as the pulling device (4) further moves towards the proximal end, the pulling device (4) is separated from the two-piece clamp (1), and the sleeve (3) is matched with a first locking mechanism (2).

16. The hemostatic clip according to claim 15, **characterized in that** the pulling device (4) comprises a rod portion (4011) and a connecting portion which are connected to each other, the connecting portion is connected to a second locking mechanism (7), and the connecting portion can be deformed and disengaged from the second locking mechanism (7) under the action of a pulling force.

17. The hemostatic clip according to claim 16, **characterized in that** the connecting portion comprises a baffle (4012) located at two sides of two clamping tail sections (1024) and an insertion plate (4013) matched with the second locking mechanism (7), and the insertion plate (4013) is inserted into the second locking mechanism (7), so that the two clamping tail sections (1024) are closed.

18. The hemostatic clip according to claim 17, **characterized in that** the pulling device (4) is composed of two pulling plates (401) that sandwich the clamping tail sections (1024), each pulling plate (401) comprises a rod portion (4011), a baffle (4012), and an insertion plate (4013), and an arrangement direction of the two pulling plates (401) is the same as an arrangement direction of two clamping pieces (102).

19. The hemostatic clip according to claim 18, **characterized in that** the two insertion plates (4013) are in a parallel arrangement, in a V-shaped arrangement, in a herringbone arrangement or in a splayed arrangement.

20. The hemostatic clip according to claim 16, **characterized in that** the connecting portion further comprises a limiting plate (8) extending towards a direction of the fixing member (101), the limiting plate (8) is provided with an opening and the opening faces towards the fixing member (101), and the fixing member (101) is in clearance fit with the limiting plate (8).
